Europäisches Patentamt

European Patent Office

Office européen des brevets

(11) Veröffentlichungsnummer: **0 134 307**
**B1**

(12) **EUROPÄISCHE PATENTSCHRIFT**

(45) Veröffentlichungstag der Patentschrift:
19.04.89

(51) Int. Cl.⁴: **C 08 J 7/04,** C 07 K 17/08,
C 12 N 11/08, G 01 N 33/545

(21) Anmeldenummer: 83111144.8

(22) Anmeldetag: 08.11.83

(54) Voraktivierte Kunststoffoberflächen zur Immobilisierung von organisch-chemischen und biologischen Materialien, Verfahren zur Herstellung und Verwendung derselben.

(30) Priorität: 29.07.83 DE 3327327

(43) Veröffentlichungstag der Anmeldung:
20.03.85 Patentblatt 85/12

(45) Bekanntmachung des Hinweises auf die Patenterteilung:
19.04.89 Patentblatt 89/16

(84) Benannte Vertragsstaaten:
AT BE CH DE FR GB IT LI LU NL SE

(56) Entgegenhaltungen:
EP-A-0 101 912
EP-A-0 132 117

INFECTION AND IMMUNITY, Band 38, Nr. 3,
Dezember 1982, Seiten 1203-1207; M. LEINONEN et
al.: "Class-specific antibody response to group B
Neisseria meningitidis capsular polysaccharide:
use of polylysine precoating in an enzyme-linked
immunosorbent assay"

(73) Patentinhaber: **Henning Berlin GmbH Chemie und
Pharmawerk, Komturstrasse 19- 20, D-1000 Berlin
42 (DE)**

(72) Erfinder: **Gadow, André, Dipl.- Chem.,
Schwartauer Allee 195, D-2400 Lübeck (DE)**
Erfinder: **Wood, W. Graham, Am Waldrand 29,
D-2401 Gross- Grönau (DE)**

(74) Vertreter: **Werner, Hans- Karsten, Dr.,
Deichmannhaus am Hauptbahnhof, D-5000 Köln 1
(DE)**

EP 0 134 307 B1

**Beschreibung**

Gegenstand der vorliegenden Erfindung sind voraktivierte Kunststoffoberflächen zur Immobilisierung von organisch-chemischen und biologischen Materialien wie Antigenen, Antikörpern, Allergenen, Geweben, Enzymen, Cofaktoren etc. Weiterhin betrifft die Erfindung ein Verfahren zur Herstellung solcher voraktivierter Kunststoffoberflächen sowie ihre Verwendung.

Bei vielen immunologischen, histologischen und enzymatischen Methoden ist es notwendig und zur Vereinfachung der Verfahren zweckmäßig, entsprechende Komponenten des Tests an festen Trägern zu immobilisieren. Bei Radio-, Enzym-, Fluoreszenz- und Lumineszenz-Immunoassays werden häufig immobilisierte Antikörper und Antigene verwendet. Bei histologischen Tests verwendet man immobilisiertes Gewebe. Der sog. RAST-Test (Radio-Allergo-Sorbens-Test) für die Allergendiagnostik verwendet an Papierscheibchen immobilisierte Allergene (z. B. Birkenpollen etc.). Immobilisierte Enzyme werden z. B. in sog. Enzymreaktoren verwendet, aber auch immobilisierte Enzym-Substrate und Cofaktoren werden in enzymatischen Tests, gebunden an feste Träger verwendet. Die Immobilisierung kann an verschiedenen festen Phasen wie Glas, Kunststoffen oder sonstigen homogenen Festkörpern erfolgen, wobei entweder die Gefäßwand (Röhrchen bzw. Mikrotiterplatten) oder die Oberfläche von Mikro- und Makropartikeln (Körner, Kugeln, Scheibchen etc.) eingesetzt werden. Ein beliebter Festkörper für die Immobilisierung von verschiedenen organisch-chemischen und biologischen Materialien ist Polystyrol, wobei die Kopplung an Mikro und Makropartikel erfolgen kann.

Zur besser reproduzierbaren Immobilisierung spezieller Antikörper wurden diese kovalent an Poly-Lysin gebunden, welches seinerseits an Polystyrolkugeln adsorbiert war.

Außer Polylysin sind auch andere Polyamine wie Poly-L-arginine, Poly-L-histidine oder Poly-L-ornithine untersucht worden, wobei Polylysin die besten Werte ergab; vgl. Maija Leinonen und Carl E. Frasch, Infection and Immunity, 1982, S. 1203-1207 sowie Barry M. Gray, Journal of Immunological Methods, 28 (1979) S. 187-192.

Alle bisher untersuchten Kunststoffoberflächen zur Immobilisierung von Antikörpern oder Antigenen weisen noch erhebliche Nachteile auf, da sie entweder zu nicht reproduzierbaren Werten führen oder aber zu unerwünschten schlecht kontrollierbaren Auswaschreaktionen oder unspezifischen Veränderungen der Antikörper oder Antigene.

Die Erfindung hat sich die Aufgabe gestellt, ein Verfahren zur Voraktivierung von Kunststoffoberflächen zu entwickeln, das geeignet ist, organisch-chemische und biologische Materialien fest und reproduzierbar ohne die bekannten Nachteile bisher bestehender Verfahren an Kunststoffen zu immobilisieren. Umfangreiche und die verschiedensten Parameter wie Temperatur, Reaktionsdauer, pH-Wert und Puffersystem berücksichtigende Untersuchungen bezüglich der Optimierung der Eigenschaften haben zu dem überraschenden Ergebnis geführt, daß diese Aufgabe gelöst werden kann durch Kunststoffoberflächen, die mit einem Polypeptid beschichtet werden, das aus einem definierten Copolymerisat von hydrophoben Aminosäuren wie Phenylalanin, Valin, Leucin usw. besteht sowie aus Aminosäuren mit Seitenketten die zur Aktivierung und Kopplung befähigte Gruppen tragen (Lysin, Arginin, Ornithin, Cystein, Glutaminsäure, Asparaginsäure usw.).

Typische Vertreter dieser Substanzen sind somit Polypeptide wie Poly-Phenylalanin-Lysin, Poly-Phenylalanin-Glutaminsäure, Poly-Leucin-Lysin, Poly-Valin-Cystein usw. Besonders bevorzugt sind Phenylalanin-Lysin-Copolymere.

Gegenstand der vorliegenden Erfindung sind demnach voraktivierte Kunststoffoberflächen, die mit einem Polypeptid beschichtet sind zur Immobilisierung von organisch-chemischen und biologischen Materialien, dadurch gekennzeichnet, daß das Polypeptid ein definiertes Copolymerisat von hydrophoben Aminosäuren und Aminosäuren mit zur Aktivierung und Kopplung befähigten Seitenketten ist.

Die Beschichtung kann erfindungsgemäß dadurch erfolgen, daß man die Kunststoffoberflächen mit einer Lösung oder Suspension des Polypeptids behandelt und anschließend wäscht. Im Anschluß kann dann ein Aktivierungsschritt und die Kopplung folgen.

Das verwendete Phenyl-Lysin-Copolymer weist vorzugsweise ein Molverhältnis von Phenylalanin zu Lysin von 1 : 1 auf. Prinzipiell sind jedoch auch Polymere in anderen Verhältnissen (2 : 1, 1 : 2, usw.) mit Erfolg einsetzbar.

Das Molekulargewicht der Polypeptide sollte vorzugsweise größer als 10.000 sein. Hervorragend geeignet sind Polypeptide mit einem Molekulargewicht von 30.000 bis über 200.000.

Derartige Polypeptide sind bereits im Handel und werden beispielsweise von den Firmen Miles-Yeda Ltd. (Kiryat Weizmann Rehovot, Israel) oder SIGMA Chemie GmbH, München angeboten. Niedermolekulare Polypeptide sind im allgemeinen leicht wasserlöslich, höhermolekulare sind im allgemeinen in der Wärme wasserlöslich oder noch gut suspendierbar.

Die voraktivierbaren Kunststoffoberflächen werden mit derartigen Lösungen oder Suspensionen vorzugsweise bei Raumtemperatur oder Kühlschranktemperatur einige Stunden in Berührung gebracht und danach gewaschen. Die Kunststoffoberflächen überziehen sich dabei mit einer nahezu konstanten dünnen Schicht des Polypeptids. Die hydrophoben Anteile des Polypeptides (im Falle von Poly-PhenylalaninLysin ist dies der Phenylalaninanteil) lagern sich dabei direkt auf der Kunststoffoberfläche an und die die aktivierbaren Gruppen tragenden Anteile (beispielsweise also die Lysin-Seitenkette mit der endständigen Aminogruppe), die meistens hydrophil sind, zeigen nach außen. Diese Gruppen können über in der Literatur zahlreich beschriebene Reaktionen aktiviert werden und im Anschluß an diese Aktivierung kann die Kopplung der

2

organisch-chemischen und biologischen Materialien direkt erfolgen. Die vor der Kopplung notwendige Aktivierung kann beispielsweise mit Hilfe von Kopplungsreagenzien erfolgen.

Zur Aktivierung geeigneter Kopplungsreagenzien sind beispielsweise für die direkte Kopplung Glutardialdehyd, Bromcyan, Hydrazine, Bisepoxirane, Divinylsulfone, Epichlorhydrin, Benzochinone, Trichloros-s-Triazine, Isothiocyanate, Arylamine und Phenylhydrazine. Die dabei entstehenden Bindungstypen sind beispielsweise Michael Addukte und Schiff'sche Basen, Cyanat-Ester, Triazinyle, Ether, Imidocarbonate, Amide, gemischte Anhydride, Alkylamine, Ester. Für die indirekte Kopplung kommen Reagenzien in Frage wie EEDQ (N - Ethoxycarbonyl-2-ethoxy-1,2-dihydrochinolin) Carbodiimide wie Dicyclohexylcarbodiimid, 1-Cyclohexyl-3-(2-morpholinyl-(4)-ethyl) carbodiimid-methyl-p-toluol-sulfonat, 1-Ethyl-3-(3-dimethylaminopropyl) carbodiimid hydrochlorid und N - Hydroxysuccinimide. Weiterhin kommen heterobifunktionelle Reagenzien in Frage wie MBS (m - Maleimidobenzoyl-N-hydroxysuccinimidester) und andere.

Vergleichende Untersuchungen mit anderen voraktivierten Oberflächen haben ergeben, daß erfindungsgemäß immobilisierte organisch-chemische und biologische Materialien besonders fest und vor allem mit ausgezeichneter Präzision und Reproduzierbarkeit an die Oberfläche gebunden werden, ohne daß es zu den bei den bestehenden Verfahren bekannten unspezifischen Reaktionen und Auswaschungsphänomen während späterer Verfahrensschritte kommt. Die Anwendung von sog. Detergenzien wie Tween 20 (Polyoxyethylensorbitanmonolaurat) und Triton X 100 (Polyethylenether) zur Wegwaschung von in späteren Verfahrensschritten unspezifisch gebundenen Komponenten ist problemlos möglich, ohne daß die spezifisch gekoppelten Anteile entfernt werden. Dies war bei den bestehenden rein adsorbtiven Immobilisierungsverfahren unter Verwendung von beispielsweise Polystyrol nicht möglich. Diese Vorteile wurden bei einer Vielzahl erfindungsgemäß immobilisierter organisch-chemischer und biologischer Materialien beobachtet, so daß erfindungsgemäß voraktivierte Kunststoffoberflächen bei den verschiedensten Anwendungsgebieten eingesetzt werden können.

Phenylalanin-Lysin-Copolymere gehören somit zu einer Substanzklasse die aufgrund ihrer Eigenschaften ganz allgemein zur Voraktivierung von Kunststoffoberflächen verwendet werden können, an die zur Kopplung befähigte Komponenten wie Antigene, Antikörper, Allergene, Gewebe, Enzyme, Substrate, Cofaktoren usw. gebunden werden sollen. Die Voraktivierung wirkt zuverlässig und gleichbleibend unabhängig davon mit welchen Reagenzien die endgültige Kopplung des zu immobilisierenden Materials erfolgt.

Erstmals beobachtet und beschrieben wurden diese überraschend guten Eigenschaften bei der Voraktivierung von Polystyrolkugeln mit Phenylalanin-Lysin-Copolymeren, Aktivierung mittels Glutardialdehyd und Immobilisierung von Antikörpern in der deutschen Patentanmeldung P-3 327 327.8. Es hat sich gezeigt, daß es sich hierbei um ein allgemeines und breit anwendbares Prinzip handelt. Als Kunststoffe kommen praktisch alle hydrophoben Kunststoffe in Frage wie Polystyrol, Polyethylen, Polypropylen etc.

In den nachfolgenden Beispielen sind erfindungsgemäß voraktivierte Kunststoffoberflächen sowie Verfahren zu ihrer Herstellung und der Kopplung verschiedener Materialien näher erläutert:

**Beispiel 1**

a) Immobilisierung nach Voraktivierung

5 mg Polyphenylalanin-Lysin der Firma Sigma (MG 40 KD) oder Miles-Yeda (MG 30KD) werden unter leichtem Erwärmen in insgesamt 200 ml entmineralisiertem Wasser gelöst und auf 1000 Polystyrolkugeln (Precision plastic balls, Chicago) vom Durchmesser 6,4 mm gegossen. Es wird ca. 30 min. bei Raumtemperatur unter gelegentlichem sanften Schütteln stehengelassen und dann 24 h bei Kühlschranktemperatur (4°) stehen gelassen. Die Kugeln werden dann einmal durch Überschichten mit einer 0,15 mol/l Kochsalzlösung und zweimal mit demineralisiertem Wasser gewaschen. Die so beschichteten Polystyrolkugeln können nun direkt mit einem Kopplungsreagenz umgesetzt werden, beispielsweise mit 0,5 Volumen-% Pentan-1,5-dial (Glutardialdehyd) in demineralisiertem Wasser für 30 min. bei Raumtemperatur. Nach dem letztgenannten Aktivierungsschritt können aminogruppentragende organisch-chemische und biologische Materialien direkt gekoppelt werden. Es werden im allgemeinen Mengen von 1 - 5 μg pro Polystyrolkugel angeboten. Die Immobilisierung von Antikörpern ist in den Beispielen der deutschen Patentanmeldung P 33 27 327.8 beschrieben.

b) Vergleichende Untersuchungen

Tabelle 1 und 2 zeigen die Stabilität und Bindungseigenschaften von nach Beispiel 1a immobilisierten $T_4$ (Thyroxin) sowie Antikörpern gegen h-TSH (Thyrotropin), und vergleichen sie mit den Eigenschaften einer herkömmlichen Immobilisierung. Vergleichende Übersicht der Immobilisierung von radioaktiv-markiertem [125]I-$T_4$ (Thyroxin) an Polystyrolkugeln

a) rein adsorbtiv,

b) nach Behandlung der Polystyrolkugeln mit Glutardialdehyd sowie

c) nach erfindungsgemäßer Beschichtung mit Phenylalanin-Lysin-Copolymer und anschließender Aktivierung mit Glutardialdehyd.

Kopplungsdauer 38 h bei 4° C, [125]I-$T_4$ wurde im Überschuß angeboten, gelöst in 0,01 mol/l Phosphatpuffer pH 8,5.

**Tabelle 1**

| Art der Aktivierung der Polyst. Kugeln | | gebundene Menge pro Kugel nach Waschung mit | | |
|---|---|---|---|---|
| | | 0,15 mol/l NaCl | 0,1 Vol.-% tween 20 | 5 Vol.-% KCl |
| a) | rein adsorptiv | *3688 cpm ± 669 | 513 cpm ± 54 | 973 cpm ± 109 |
| b) | nur mit Glutardialdehyd | 3097 cpm ± 366 | 344 cpm ± 110 | 1196 cpm ± 112 |
| c) | gemäß Beispiel 1a | 15615 cpm ± 254 | 10233 cpm ± 333 | 10585 cpm ± 695 |

* die angegebene Standardabweichung bezieht sich auf jeweils 10 gemessene Proben
(cpm = counts per minute)

Vergleichende Übersicht der Bindung von radioaktiv-markiertem, [125]I-TSH (humanes Thyrotropin) an Antikörper gegen humanes Thyrotropin, die unter optimierten Bedingungen
a) rein adsorptiv
b) nach Behandlung der Polystyrolkugeln mit Glutardialdehyd sowie
c) nach erfindungsgemäßer Beschichtung mit Phenylalanin-Lysin-Copolymer und anschließender Aktivierung mit Glutardialdehyd an Polystyrolkugeln gebunden wurden.

Angegeben ist das Verhältnis der spezifischen und unspezifischen Bindung zur angebotenen Totalaktivität nach einer Reaktionszeit von 6 h bei Raumtemperatur. Zur Messung der unspezifischen Bindung wurde radioaktiv-markiertes [125]I-TBG (Thyroxinbindendes Globulin) verwendet.

**Tabelle 2**

| Art der Aktivierung der Polystyrol-Kugeln | | Verhältnis Bo/T, UB/T nach Waschung mit | | | | | |
|---|---|---|---|---|---|---|---|
| | | 0,15 mol/l NaCl | | 0,1 Vol.-% tween 20 | | 5 Vol.-% KCl | |
| | | (Bo/T)x100 | (UB/T)x100 | (Bo/T)x100 | (UB/T)x100 | (Bo/T)x100 | (UB/T)x100 |
| a) | rein adsorptiv | 57 ± 2* | 2 | 19 ± 0,7 | 2 | 35 ± 0,6 | 2 |
| b) | nur mit Glutardialdehyd | 11 ± 1 | 4 | 6 ± 0,5 | 3 | 12 ± 0,2 | 4 |
| c) | gemäß Beispiel 1a | 44 ± 0,6 | 4 | 39 ± 0,4 | 3 | 39 ± 0,2 | 5 |

T = Totalaktivität;
Bo = Spezifische Bindung;
UB = unspezifische Bindung.
* Die angegebene Standardabweichung bezieht sich auf jeweils 10 gemessene Problem.

**Beispiel 2**

Polystyrolröhrchen werden mit der gleichen Lösung wie in Beispiel 1 gefüllt, über Nacht stehengelassen und 1x mit 0,15 mol/l NaCl in demin. $H_2O$ sowie 2x mit demin. $H_2O$ ausgespült. Die so behandelten Röhrchen werden mit der im Beispiel 1 beschriebenen Pentan-1,5-dial-Lösung gefüllt, 30 min. bei Raumtemperatur zur Reaktion gebracht und entsprechend gewaschen. Die so behandelten Röhrchen sind danach unmittelbar geeignet, organisch-chemische und biologische Materialien mit zur Kopplung befähigten Aminogruppen fest zu binden.

**Beispiel 3**

Die einzelnen Vertiefungen einer Mikrotiterplatte werden mit der in Beispiel 1 beschriebenen Lösung gefüllt und wie in Beispiel 2 weiterbehandelt. Die Platte bindet biologisches Material.

**Beispiel 4**

100 g Polystyrol in Mikropartikelform (Dow-Latex, Serva) werden mit der Lösung aus Beispiel 1 überschichtet. Die Weiterreaktion erfolgt in analoger Weise. Zur Waschung wird filtriert.

Bei der Aktivierung mit Glutardialdehyd und der anschließenden Kopplung von aminogruppentragenden Substanzen bilden sich Schiff'sche Basen, die unter Verwendung von Natriumborhydrid oder Natriumcyanoborhydrid reduziert werden können. Die erfindungsgemäße Beschichtung übersteht solche Behandlungen ohne Beeinträchtigungen.

**Beispiel 5**

Röhrchen aus Polyethylen und Polypropylen werden wie im Beispiel 2 beschrieben voraktiviert. Die so behandelten Röhrchen sind in gleicher Weise wie Polystyrolröhrchen geeignet, organisch-chemische und biologische Materialien mit zur Kopplung befähigten Aminogruppen fest zu binden.

**Patentansprüche**

1. Voraktivierte Kunststoffoberflächen die mit einem Polypeptid beschichtet sind zur Immobilisierung von organisch-chemischen und biologischen Materialien, dadurch gekennzeichnet, daß das Polypeptid ein definiertes Copolymerisat von hydrophoben Aminosäuren und Aminosäuren mit zur Aktivierung und Kopplung befähigten Seitenketten ist.

2. Voraktivierte Kunststoffoberflächen gemäß Anspruch 1, dadurch gekennzeichnet, daß die Oberflächen mit Phenylalanin-Lysin-Copolymer beschichtet sind.

3. Verfahren zur Voraktivierung von Kunststoffoberflächen für die Immobilisierung von organisch-chemischen und biologischen Materialien, dadurch gekennzeichnet, daß die Kunststoffoberfläche mit einer Lösung oder Suspension eines Polypeptids, das ein definiertes Copolymerisat von hydrophoben Aminosäuren und Aminosäuren mit zur Aktivierung und Kopplung befähigten Seitenketten ist, behandelt und anschließend gewaschen wird.

4. Verfahren gemäß Anspruch 3, dadurch gekennzeichnet, daß als Polypeptid ein Phenylalanin-Lysin-Copolymer verwendet wird.

5. Verwendung von voraktivierten Kunststoffoberflächen gemäß Ansprüchen 1 und 2 zur Immobilisierung von organisch-chemischen und biologischen Materialien.

6. Verwendung von voraktivierten Kunststoffoberflächen gemäß Anspruch 5, dadurch gekennzeichnet, daß als biologisches Material Antikörper oder Antigene immobilisiert werden.

**Claims**

1. Pre-activated surfaces of synthetic materials coated with a polypeptide for immobilizing organochemical and biological materials, characterized in that the polypeptide is a defined copolymer of hydrophobic amino acids and amino acids having side chains capable of activation and coupling.

2. Pre-activated surfaces of synthetic materials according to claim 1, characterized in that the surfaces have been coated with phenylalanine-lysine copolymer.

3. A process for the pre-activation surfaces of synthetic materials for immobilizing organochemical and biological materials, characterized in that the surface of the synthetic material is treated with a solution or suspension of polypeptide which is a defined copolymer of hydrophobic amino acids and amino acids having side chains capable of activation and coupling, and is subsequently washed.

4. The process according to claim 3, characterized in that a phenylalanine-lysine copolymer is used as the polypeptide.

5. Use of pre-activated surfaces of synthetic materials according to claims 1 and 2 for immobilizing organochemical and biological materials.

6. Use of pre-activated surfaces of synthetic materials according to claim 5, characterized in that antibodies or antigens are immobilized as biological material.

**Revendications**

1. Surfaces de matières synthétiques préactivées qui sont revêtues d'un polypeptide pour l'immobilisation de matières organochimiques et biologiques, caractérisées en ce que le polypeptide est un copolymère défini d'acides aminés hydrophobes et d'acides aminés à chaînes latérales aptes à l'activation et au couplage.

2. Surfaces de matières synthétiques préactivées selon la revendication 1, caractérisées en ce que les surfaces sont revêtues de copolymères de phénylalanine-lysine.

3. Procédé de préactivation de surfaces de matières synthétiques pour l'immobilisation de matières organochimiques et biologiques, caracterisé en ce que la surface de matières synthétiques est traitée avec une solution ou une suspension d'un polypeptide, qui est un copolymère défini d'acides aminés hydrophobes et d'acides aminés à chaînes latérales aptes à l'activation et au couple et que ce traitement est suivi d'un lavage.

4. Procédé selon la revendication 3, caractérisé en ce que l'on emploie comme polypeptide un copolymère de phénylalanine-lysine.

5. Emploi de surfaces de matières synthétiques préactivées selon les revendications 1 et 2 pour l'immobilisation de matières organochimiques et biologiques.

6. Emploi de surfaces de matières synthétiques préactivées selon la revendication 5, caractérisé en ce que comme matière biologique on inmobilise des anticorps ou des antigènes